# EUROPEAN PATENT APPLICATION

(11) **EP 4 745 249 A1**
(43) Date of publication of application: **20.05.2026**
(21) Application number: 24843094.4
(22) Date of filing: 12.07.2024
(51) Int. Cl.: C12Q 1/6881

(54) **ONE OR MORE PRIMERS FOR HLA-DP GENES**

(30) Priority: 14.07.2023 JP 2023115719
(71) Applicant: Japan Institute for Health Security, Tokyo 162-8655 (JP); H.U. Group Research Institute G.K., Akiruno-shi, Tokyo 197-0833 (JP)
(72) Inventor: TOKUNAGA, Katsushi, Tokyo 162-8655 (JP); KHOR, Seik-Soon, Tokyo 162-8655 (JP); OMAE, Yosuke, Tokyo 162-8655 (JP); NAITO, Ikue, Akiruno-shi, Tokyo 197-0833 (JP); SATO, Tetsuya, Akiruno-shi, Tokyo 197-0833 (JP); WAKABAYASHI, Shunichi, Akiruno-shi, Tokyo 197-0833 (JP); TANAKA, Kosei, Akiruno-shi, Tokyo 197-0833 (JP)
(74) Representative: Müller-Boré & Partner Patentanwälte PartG mbB
(86) International application number: PCT/JP2024/025312
(87) International publication number: WO 2025/018304

(57) **Abstract**

The present invention provides one or more kinds of primers capable of highly comprehensively detecting or amplifying an HLA allele for the full-length of an HLA-DP gene (comprising an untranslated region). More specifically, the present invention provides one or more kinds of HLA-DP gene primers selected from the group consisting of the following (1) and (2), and a combination thereof:
(1) one or more kinds of HLA-DPA1 gene primers, which are
a primer comprising a nucleotide sequence of SEQ ID NO:1 or the like, or a nucleotide sequence complementary thereto, or the like; and
(2) one or more kinds of HLA-DPB1 gene primers, which are
a primer comprising a nucleotide sequence of SEQ ID NO:9 or the like, or a nucleotide sequence complementary thereto, or the like.

## Description

### FIELD

The present invention relates to one or more kinds of HLA-DP gene primers and the like.

### BACKGROUND

A human leukocyte antigen (HLA) region on a short arm of chromosome 6 indicates very high polymorphism. Due to this high polymorphism, the HLA region is to be tested as a main application antigen in transplantation and blood transfusion, and has a function of presenting various exogeneous or autologous antigens.

HLA molecules are roughly classified into class I molecules such as HLA-A, HLA-B, and HLA-C and class II molecules such as HLA-DR, HLA-DQ, and HLA-DP from a viewpoint of functions and structures. The class II molecules comprise α chain and β chain, is expressed mainly on dendritic cells, macrophages, and B cells which are antigen-presenting cells, and presents peptides derived from exogeneous antigens.

In an HLA class II gene region, an HLA-DR gene and an HLA-DQ gene are in a strong linkage disequilibrium relationship, whereas a recombination hot spot is present between the HLA-DQ gene and an HLA-DP gene.

The α chain and the β chain of the HLA-DP molecule are encoded by an HLA-DPA1 gene and an HLA-DPB1 gene, respectively, and both the α chain and the β chain exhibit polymorphism. The HLA-DPA1 gene and the HLA-DPB1 gene are arranged on a genome as a head-to-head gene pair, and are characterized in that a bidirectional transcriptional promoter simultaneously controls the two genes (Non-Patent Literature 1). In addition, it has been reported that SNP (rs9277534) in a 3'-untranslated region of the HLA-DPB1 gene is associated with an mRNA expression level of the HLA-DP gene (Non-Patent Literature 2).

A commercially available HLA allele typing kit is intended only for coding regions of the HLA-DPA1 gene and the HLA-DPB1 gene, and cannot perform sequence analysis of a full-length gene comprising an untranslated region. Therefore, for the full-length gene sequence comprising an untranslated region, only some alleles are registered in an IMGT/HLA database. In the future, sequence analysis of the full-length of HLA-DPA1 gene and the full-length of HLA-DPB1 gene comprising an untranslated region is important.

In addition, some primers capable of amplifying the HLA-DPA1 gene and the HLA-DPB1 gene by long range PCR have been reported in prior art. However, there has not been reported a primer set capable of comprehensively amplifying a large number of HLA alleles by one PCR for a full-length gene comprising an untranslated region. In particular, prior art has not reported a primer set capable of comprehensively amplifying HLA alleles by one PCR for a full-length gene comprising an untranslated region and capable of phasing HLA-DP. For example, in primers described in Patent Literatures 1 and 2 and Non-Patent Literature 1, comprehensiveness of a large number of HLA alleles has not been studied. Disclosure of these Literatures is as follows.

The HLA-DPB1 gene primer described in Patent Literature 1 cannot amplify a full-length gene by PCR. In addition, since the method of Patent Literature 1 is a sequence analysis method which uses PCR for three segmented regions of coding regions of an HLA-DPA1 gene and an HLA-DPB1 gene and an intergenic region thereof, it is necessary to align six kinds of sequences in order to obtain a phased sequence from the HLA-DPA1 gene to the HLA-DPB1 gene. Therefore, an allele and a promoter sequence cannot be phased by PCR at one time.

In addition, the HLA-DPA1 gene primer described in Patent Literature 2 does not contain a 5'-untranslated region, and therefore cannot amplify a full-length gene. In addition, the HLA-DPB1 gene primer described in Patent Literature 2 cannot amplify a full-length gene by PCR at one time.

Furthermore, the method of Non-Patent Literature 1 needs to use three kinds of primer sets (a total of six primers) in order to phase HLA-DP.

### PRIOR ART LITERATURE

### PATENT LITERATURE

Patent Literature 1: JP 2016-10318 A
Patent Literature 2: JP 2017-158570 A

### NON-PATENT LITERATURE

Non-Patent Literature 1: Truong L et al., HLA. 2020; 96(3): 299-311.
Non-Patent Literature 2: Petersdorf EW et al., N Engl J Med. 2015; 373(7): 599-609.

### SUMMARY

### TECHNICAL PROBLEM

An object of the present invention is to provide one or more kinds of primers capable of highly comprehensively detecting or amplifying an HLA allele for the full-length of an HLA-DP gene (comprising an untranslated region).

Another object of the present invention is to provide a primer set capable of comprehensively amplifying HLA alleles by a gene amplification method at one time for the full-length of an HLA-DP gene (comprising an untranslated region) and capable of phasing the HLA-DP gene more easily than a conventional method.

### SOLUTION TO PROBLEM

As a result of intensive studies, the present inventors have found one or more kinds of HLA-DP gene primers capable of highly comprehensively detecting or amplifying an HLA allele for the full-length of an HLA-DP gene (comprising an untranslated region). In fact, the one or more kinds of HLA-DP gene primers found by the present inventors have been experimentally confirmed to be able to highly comprehensively amplify a large number of HLA allele groups comprising a putative novel HLA allele (Tables 7 to 9). On the other hand, the primers described in Patent Literatures 1 and 2 and Non-Patent Literature 1 are construed to comprise a large number of mismatches because comprehensiveness of a large number of HLA alleles has not been studied. In fact, in the HLA-DPB1 gene primer described in Patent Literature 1, it is thought that there is a high possibility that PCR amplification is not observed or uniform amplification is affected because there are mismatches in 19 alleles (mismatch: about 7%) in 137 cases (number of alleles: 274) described in EXAMPLES (see EXAMPLES). On the basis of such findings, the present inventors have succeeded in providing one or more kinds of HLA-DP gene primers, thereby completing the present invention.

That is, the present invention is as follows.
[1] One or more kinds of HLA-DP gene primers selected from the group consisting of the following (1) and (2), and a combination thereof:
   (1) one or more kinds of HLA-DPA1 gene primers, which are
   (1a) a first primer comprising a nucleotide sequence of SEQ ID NO:1 or SEQ ID NO:5, or a nucleotide sequence complementary thereto and/or (1b) a second primer comprising a nucleotide sequence of SEQ ID NO:3 or SEQ ID NO:7, or a nucleotide sequence complementary thereto; and
   (2) one or more kinds of HLA-DPB1 gene primers, which are
   (2a) a first primer comprising a nucleotide sequence of SEQ ID NO:9, SEQ ID NO:13, or SEQ ID NO:17, or a nucleotide sequence complementary thereto and/or (2b) a second primer comprising a nucleotide sequence of SEQ ID NO:11 or SEQ ID NO:15, or a nucleotide sequence complementary thereto.
[2] The one or more kinds of HLA-DP gene primers according to [1], which are an HLA-DP gene amplification primer set selected from the group consisting of the following (1') and (2'), and a combination thereof:
   (1') an HLA-DPA1 gene amplification primer set, which is
      a primer set comprising (1a) a first primer comprising a nucleotide sequence of SEQ ID NO:1 or SEQ ID NO:5, or a nucleotide sequence complementary thereto, and (1b) a second primer comprising a nucleotide sequence of SEQ ID NO:3 or SEQ ID NO:7, or a nucleotide sequence complementary thereto; and
   (2') an HLA-DPB1 gene amplification primer set, which is
      a primer set comprising (2a) a first primer comprising a nucleotide sequence of SEQ ID NO:9, SEQ ID NO:13, or SEQ ID NO:17, or a nucleotide sequence complementary thereto, and (2b) a second primer comprising a nucleotide sequence of SEQ ID NO:11 or SEQ ID NO:15, or a nucleotide sequence complementary thereto.
[3] The one or more HLA-DP gene primers according to [1], wherein
   (1a) the first primer comprising a nucleotide sequence of SEQ ID NO:1 or SEQ ID NO:5, or a nucleotide sequence complementary thereto is (1a') a first primer comprising a nucleotide sequence of SEQ ID NO:2 or SEQ ID NO:6, or a nucleotide sequence complementary thereto,
   (1b) the second primer comprising a nucleotide sequence of SEQ ID NO:3 or SEQ ID NO:7, or a nucleotide sequence complementary thereto is (1b') a second primer comprising a nucleotide sequence of SEQ ID NO:4 or SEQ ID NO:8, or a nucleotide sequence complementary thereto,
   (2a) the first primer comprising a nucleotide sequence of SEQ ID NO:9, SEQ ID NO:13, or SEQ ID NO:17, or a nucleotide sequence complementary thereto is (2a') a first primer comprising a nucleotide sequence of SEQ ID NO:10, SEQ ID NO:14, or SEQ ID NO:18, or a nucleotide sequence complementary thereto, and
   (2b) the second primer comprising a nucleotide sequence of SEQ ID NO:11 or SEQ ID NO:15, or a nucleotide sequence complementary thereto is (2b') a second primer comprising a nucleotide sequence of SEQ ID NO:12 or SEQ ID NO:16, or a nucleotide sequence complementary thereto.
[4] A method for detecting an HLA-DP gene, comprising:
   detecting one or more kinds of HLA-DP genes in a sample obtained from a human subject using the one or more kinds of HLA-DP gene primers according to any of [1] to [3],
   wherein the one or more kinds of HLA-DP genes are selected from the group consisting of an HLA-DPA1 gene, an HLA-DPB1 gene, and a combination thereof.
[5] The method according to [4], wherein the one or more kinds of HLA-DP genes are detected by amplifying the one or more kinds of HLA-DP genes.
[6] A reagent for detecting an HLA-DP gene, comprising the one or more kinds of HLA-DP gene primers according to any of [1] to [3].
[7] A kit for detecting an HLA-DP gene, comprising:
   (a) the one or more kinds of HLA-DP gene primers according to any of [1] to [3]; and
   (b) a polymerase.

### EFFECTS OF INVENTION

According to the present invention, HLA alleles of an HLA-DP gene can be highly comprehensively detected or amplified. In addition, according to the present invention, a full-length gene comprising an untranslated region of the HLA-DP gene can be amplified. Furthermore, according to the present invention, an HLA-DPA1 gene and an HLA-DPB1 gene can be phased with two kinds of primer sets.

### BRIEF DESCRIPTION OF DRAWINGS

FIG. 1 is a diagram illustrating the summary of a relationship between HLA-DP genes (HLA-DPA1 and HLA-DPB1 genes) and SNP (rs9277534), and the primers of the present invention.
FIG. 2 is a diagram illustrating the results of PCR amplification of an HLA-DPA1 gene. The numbers correspond to conditions presented in Table 4. M: molecular weight marker; D: Genomic DNA extracted from a cell line established from an African population (Daudi) was used as a template; L: Genomic DNA extracted from Hispanic American-derived human peripheral blood mononuclear cells was used as a template; N: No genomic DNA was used (negative control).
FIG. 3 is a diagram illustrating the results of PCR amplification of an HLA-DPB1 gene. M: molecular weight marker; D: Genomic DNA extracted from a cell line established from an African population (Daudi) was used as a template; L: Genomic DNA extracted from Hispanic American-derived human peripheral blood mononuclear cells was used as a template; N: No genomic DNA was used (negative control).
FIG. 4 is a diagram illustrating novel HLA alleles detected with the primers of the present invention.

### EMBODIMENTS FOR CARRYING OUT INVENTION

The present invention provides one or more kinds of HLA-DP gene primers selected from the group consisting of the following (1) and (2), and a combination thereof:
(1) one or more kinds of HLA-DPA1 gene primers, which are
(1a) a first primer comprising a nucleotide sequence of SEQ ID NO:1 or SEQ ID NO:5, or a nucleotide sequence complementary thereto and/or (1b) a second primer comprising a nucleotide sequence of SEQ ID NO:3 or SEQ ID NO:7, or a nucleotide sequence complementary thereto; and
(2) one or more kinds of HLA-DPB1 gene primers, which are
(2a) a first primer comprising a nucleotide sequence of SEQ ID NO:9, SEQ ID NO:13, or SEQ ID NO:17, or a nucleotide sequence complementary thereto and/or (2b) a second primer comprising a nucleotide sequence of SEQ ID NO:11 or SEQ ID NO:15, or a nucleotide sequence complementary thereto.

The one or more kinds of primers of the present invention are not particularly limited as long as they can be annealed to a target site of an HLA-DPA1 or an HLA-DPB1 gene. For example, as the one or more kinds of primers of the present invention, any primer containing a natural nucleic acid or an artificial nucleic acid can be used, but a DNA primer is preferable from a viewpoint of versatility, cost, and the like.

In one embodiment, the one or more kinds of primers of the present invention may be one kind of HLA-DP gene primer. For example, such one or more kinds of primers of the present invention are useful as sequencing primers or nucleic acid molecules directed to a target of an RNA-guided nuclease (Example, Cas9) because of having excellent HLA allele comprehensiveness.

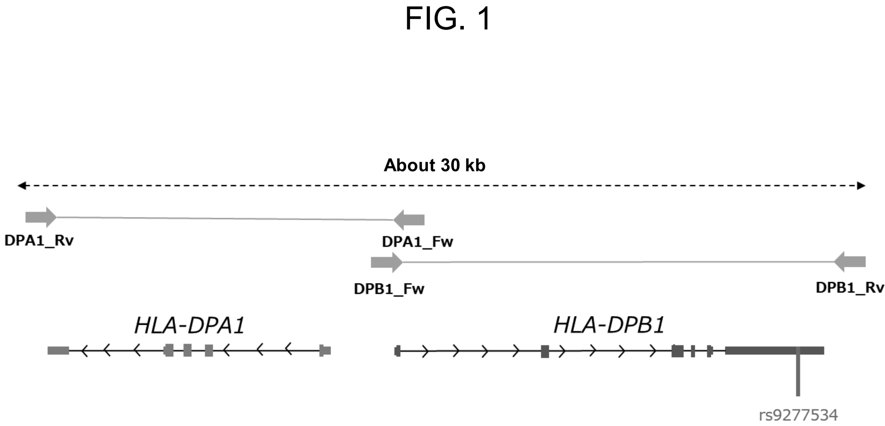

When the one or more kinds of primers of the present invention are sequencing primers or nucleic acid molecules directed to a target of an RNA-guided nuclease, one kind of primer is the following primer from a viewpoint of targeting a region in an HLA-DP gene (FIG. 1).
(1) one or more kinds of HLA-DPA1 gene primers, which are
(1a) a first primer comprising a nucleotide sequence of SEQ ID NO:1 or SEQ ID NO:5 or (1b) a second primer comprising a nucleotide sequence of SEQ ID NO:3 or SEQ ID NO:7; or
(2) one or more kinds of HLA-DPB1 gene primers, which are
(2a) a first primer comprising a nucleotide sequence of SEQ ID NO:9, SEQ ID NO:13, or SEQ ID NO:17 or (2b) a second primer comprising a nucleotide sequence of SEQ ID NO:11 or SEQ ID NO:15.

When the one or more kinds of primers of the present invention are sequencing primers or nucleic acid molecules directed to a target of an RNA-guided nuclease, one kind of primer may be (1a) a first primer comprising a nucleotide sequence of SEQ ID NO:1 or SEQ ID NO:5, or (2a) a first primer comprising a nucleotide sequence of SEQ ID NO:9, SEQ ID NO:13, or SEQ ID NO:17 from a viewpoint of targeting an untranslated region upstream of a gene that is particularly important for gene expression (FIG. 1).

In another embodiment, the one or more kinds of primers of the present invention may be a primer set comprising two or more kinds of primers. For example, such a primer set is excellent in HLA allele comprehensiveness, and therefore is useful as a gene amplification primer set.

When the one or more kinds of primers of the present invention are a primer set comprising two or more kinds of primers, the one or more kinds of primers may be the following primer set comprising at least one kind of specific primer excellent in HLA allele comprehensiveness.
(1) a primer set comprising two or more kinds of HLA-DPA1 gene amplification primers, which is
   a primer set comprising (1a) a first primer comprising a nucleotide sequence of SEQ ID NO:1 or SEQ ID NO:5, or a nucleotide sequence complementary thereto, and (1b) a second primer comprising a nucleotide sequence of SEQ ID NO:3 or SEQ ID NO:7, or a nucleotide sequence complementary thereto, or
   a primer set comprising a primer selected from (1a) the first primer comprising a nucleotide sequence of SEQ ID NO:1 or SEQ ID NO:5, or a nucleotide sequence complementary thereto or (1b) the second primer comprising a nucleotide sequence of SEQ ID NO:3 or SEQ ID NO:7, or a nucleotide sequence complementary thereto, and another primer capable of amplifying an HLA-DPA1 gene in a combination with the selected primer; or
(2) a primer set comprising two or more kinds of HLA-DPB1 gene amplification primers, which is
   a primer set comprising (2a) a first primer comprising a nucleotide sequence of SEQ ID NO:9, SEQ ID NO:13, or SEQ ID NO:17, or a nucleotide sequence complementary thereto, and (2b) a second primer comprising a nucleotide sequence of SEQ ID NO:11 or SEQ ID NO:15, or a nucleotide sequence complementary thereto, or
   a primer set comprising a primer selected from (2a) the first primer comprising a nucleotide sequence of SEQ ID NO:9, SEQ ID NO:13, or SEQ ID NO:17, or a nucleotide sequence complementary thereto or (2b) the second primer comprising a nucleotide sequence of SEQ ID NO:11 or SEQ ID NO:15, or a nucleotide sequence complementary thereto, and another primer capable of amplifying an HLA-DPB1 gene in a combination with the selected primer.

The number of primers required for gene amplification varies depending on the type of gene amplification method. For example, in PCR, the number of primers required for gene amplification is 2. Meanwhile, in LAMP (loop-mediated isothermal amplification) (see, e.g., WO00/28082A), the number of primers required for gene amplification is 4 or 6. Therefore, when amplification by a gene amplification method requiring more than two primers is intended, the primer set may include an additional primer.

Preferably, the one or more kinds of primers of the present invention may be the following primer set comprising two kinds of specific primers.
(1') an HLA-DPA1 gene amplification primer set, which is
   a primer set comprising (1a) a first primer comprising a nucleotide sequence of SEQ ID NO:1 or SEQ ID NO:5, or a nucleotide sequence complementary thereto, and (1b) a second primer comprising a nucleotide sequence of SEQ ID NO:3 or SEQ ID NO:7, or a nucleotide sequence complementary thereto; and/or
(2') an HLA-DPB1 gene amplification primer set, which is
   a primer set comprising (2a) a first primer comprising a nucleotide sequence of SEQ ID NO:9, SEQ ID NO:13, or SEQ ID NO:17, or a nucleotide sequence complementary thereto, and (2b) a second primer comprising a nucleotide sequence of SEQ ID NO:11 or SEQ ID NO:15, or a nucleotide sequence complementary thereto.

In the above primer sets (1') and (2'), two kinds of specific primers are used. These two kinds of specific primers are designed so as to generate an amplification product having a large size of 14000 bp or more (EXAMPLES). Therefore, the above primer sets (1') and (2') are preferably used in a gene amplification method capable of easily generating an amplification product having a large size. Examples of such a gene amplification method include PCR, SDA (STRAND Displacement Amplification), Hybrid Capture, LCR (Ligase Chain Reaction), and Cleavase Invader.

In a case where the one or more kinds of primers of the present invention are a primer set comprising the above two kinds of specific primers, it is possible to amplify an HLA allele of a target gene more highly comprehensively as compared with a case where the one or more kinds of primers of the present invention are a primer set comprising one kind of specific primer, and it is also possible to amplify a full-length gene of the gene comprising an untranslated region.

A primer comprising SEQ ID NO:1, 3, 5, 7, 9, 11, 13, 15, or 17, or a nucleotide sequence complementary thereto may have an additional nucleotide(s) added to 5'-end or 3'-end thereof. Examples of such a nucleotide(s) include a nucleotide complementary to a corresponding nucleotide(s) at a target site. The number of the complementary nucleotides is not particularly limited, and may be, for example, 1 to 10, and preferably 1 to 5. When such a nucleotide(s) is added to the primer, annealing to the target site can be performed more stably, and detection or amplification efficiency can be improved. However, the primer comprising SEQ ID NO:1, 3, 5, 7, 9, 11, 13, 15, or 17, or a nucleotide sequence complementary thereto, itself can be annealed well to the target site, and therefore does not have to have an additional nucleotide(s) added to 5'-end or 3'-end thereof. In addition, the primer may be chemically modified (e.g., modified with an amino group), or may have a linker added.

In a certain embodiment, the first and second primers each having an additional nucleotide(s) added to 5'-end or 3'-end thereof may be as follows.

The first primer comprising a nucleotide sequence of SEQ ID NO:1 or a nucleotide sequence complementary thereto may be a first primer comprising a nucleotide sequence of SEQ ID NO:2 or a nucleotide sequence complementary thereto.

The second primer comprising a nucleotide sequence of SEQ ID NO:3 or a nucleotide sequence complementary thereto may be a second primer comprising a nucleotide sequence of SEQ ID NO:4 or a nucleotide sequence complementary thereto.

The first primer comprising a nucleotide sequence of SEQ ID NO:5 or a nucleotide sequence complementary thereto may be a first primer comprising a nucleotide sequence of SEQ ID NO:6 or a nucleotide sequence complementary thereto.

The second primer comprising a nucleotide sequence of SEQ ID NO:7 or a nucleotide sequence complementary thereto may be a second primer comprising a nucleotide sequence of SEQ ID NO:8 or a nucleotide sequence complementary thereto.

The first primer comprising a nucleotide sequence of SEQ ID NO:9 or a nucleotide sequence complementary thereto may be a first primer comprising a nucleotide sequence of SEQ ID NO:10 or a nucleotide sequence complementary thereto.

The second primer comprising a nucleotide sequence of SEQ ID NO:11 or a nucleotide sequence complementary thereto may be a second primer comprising a nucleotide sequence of SEQ ID NO:12 or a nucleotide sequence complementary thereto.

The first primer comprising a nucleotide sequence of SEQ ID NO:13 or a nucleotide sequence complementary thereto may be a first primer comprising a nucleotide sequence of SEQ ID NO:14 or a nucleotide sequence complementary thereto.

The second primer comprising a nucleotide sequence of SEQ ID NO:15 or a nucleotide sequence complementary thereto may be a second primer comprising a nucleotide sequence of SEQ ID NO:16 or a nucleotide sequence complementary thereto.

The first primer comprising a nucleotide sequence of SEQ ID NO:17 or a nucleotide sequence complementary thereto may be a first primer comprising a nucleotide sequence of SEQ ID NO:18 or a nucleotide sequence complementary thereto.

An summary of the nucleotide sequences of SEQ ID NO:1 to SEQ ID NO:18 is as follows (for details, see Table 4 and <Identification of core sequence> in EXAMPLES).

**Table 1. Summary of nucleotide sequences of SEQ ID NO:1 to SEQ ID NO:18**

| Target | Type | Primer sequence (5' → 3') | SEQ ID NO: |
|---|---|---|---|
| HLA-DPA1 | FW1 | **AACCTATCCCCCTCCCT** | 1 |
| | | A**AACCTATCCCCCTCCCT**CC | 2 |
| | RV1 | **ATCTGACCTGGAAA** | 3 |
| | | GCC**ATCTGACCTGGAAA**CCA | 4 |
| | FW2 | **ACTGTACCCATGTC** | 5 |
| | | TTT**ACTGTACCCATGT**CCTTGGAG | 6 |
| | RV2 | **CAGAACCCAGAAAGATT** | 7 |
| | | CCT**CAGAACCCAGAAAGATT**CAGA | 8 |
| HLA-DPB1 | FW1 | **TCAGATGCTTTGAAGG** | 9 |
| | | AGAA**TCAGATGCTTTGAAGG**AGGT | 10 |
| | RV1 | **GCCAGAGGTCTCATTA** | 11 |
| | | GAGTCTT**GCCAGAGGTCTCATTAA** | 12 |
| | FW2 | **CGCCCATATTTTGAT** | 13 |
| | | TG**CGCCCATATTTTGAT**CTTGTTT | 14 |
| | RV2 | **GAACTAGGGGACA** | 15 |
| | | ATTGT**GAACTAGGGGACA**TTTGGA | 16 |
| | FW3 | **AGAGAGGCAGTGTCG** | 17 |
| | | CCA**AGAGAGGCAGTGTCG**TT | 18 |

| | | | |
|---|---|---|---|
| FW: Forward primer RV: Reverse primer | | | |

A primer comprising SEQ ID NO:2, 4, 6, 8, 10, 12, 14, 16, or 18, or a nucleotide sequence complementary thereto may have an additional nucleotide(s) added to 5'-end or 3'-end thereof. Examples of such a nucleotide(s) include a nucleotide(s) complementary to a nucleotide(s) at a target site. The number of the complementary nucleotide(s) is not particularly limited, and may be, for example, 1 to 10, and preferably 1 to 5. When such a nucleotide(s) is added to the primer, annealing to the target site can be performed more stably, and detection or amplification efficiency can be improved. However, the primer comprising SEQ ID NO:2, 4, 6, 8, 10, 12, 14, 16, or 18, or a nucleotide sequence complementary thereto, itself can be annealed very well to the target site, and therefore does not have to have an additional nucleotide added to 5'-end or 3'-end thereof. In addition, the primer may be chemically modified (e.g., modified with an amino group), or may have a linker added.

In a primer comprising SEQ ID NO:1 to SEQ ID NO:18 or a nucleotide sequence complementary thereto, a functional moiety such as a labeling moiety that enables identification (e.g., non-complementary nucleotide-containing nucleotide sequences such as a barcode sequence) may be added to 5'-end thereof. Examples of such a functional moiety include Universal Sequence (PacBio) and Adapter Overhang Nucleotide Sequence (Illumina).

The primer as described above used in the present invention can be designed so as to have a specific number of nucleotides (nucleotide length). Such number of nucleotides may be, for example, 15 or more, preferably 16 or more, more preferably 17 or more, still more preferably 18 or more, and particularly preferably 20 or more. Such number of nucleotides may be, for example, 50 or less, preferably 45 or less, more preferably 40 or less, still more preferably 35 or less, and particularly preferably 30 or less. More specifically, such number of nucleotides may be, for example, 15 to 50, preferably 16 to 45, more preferably 17 to 40, still more preferably 18 to 35, and particularly preferably 20 to 30.

The present invention also provides a method for detecting an HLA-DP gene. The method of the present invention includes detecting one or more kinds of HLA-DP genes selected from the group consisting of an HLA-DPA1 gene, an HLA-DPB1 gene, and a combination thereof in a sample obtained from a human subject using the one or more kinds of primers of the present invention.

As the sample obtained from a human subject, any sample containing a target of the one or more kinds of primers of the present invention can be used. Therefore, the sample obtained from a human subject is not particularly limited as long as the sample contains a genome and/or a transcript of a target HLA-DP gene, and examples thereof include a sample collected from a human and a sample prepared from a sample collected from a human (e.g., a cell sample). Preferably, a genome-containing sample is used as the sample. The genome-containing sample is preferably a low-invasive sample. Examples of such a sample include hair, saliva, blood (e.g., whole blood, plasma and serum), and mucosa (e.g., oral mucosa and nasal mucosa) .

For detection of an HLA-DP gene in a sample, a genomic DNA or a transcript of an HLA-DP gene may be extracted from the sample. Such extraction can be performed by any method. The extracted genomic DNA or transcript of an HLA-DP gene can be subjected to an appropriate gene amplification method (e.g., PCR or LAMP) according to the size of an intended amplification product. Alternatively, the sample may be directly subjected to a gene amplification method (e.g., direct PCR).

In the method of the present invention, an amplification product obtained by the gene amplification method using the gene amplification primer set of the present invention may be sequenced by a long read sequencer. Examples of the long read sequencer include a Sequel system (Pacific Biosciences of California, Inc.) and a nanopore sequencer (Oxford Nanopore Technologies plc.). The gene amplification primer set of the present invention can amplify a full-length gene of an HLA-DP gene comprising an untranslated region. By determining a nucleotide sequence of this amplification product by a long read sequencer, full-length genes of an HLA-DPA1 gene and an HLA-DPB1 gene comprising an untranslated region can be analyzed, and an HLA-DP region can be phased for each allele.

The method of the present invention is useful for, for example, transplantation, selection of a cancer therapeutic agent/therapeutic method (e.g., a cancer vaccine), or HLA examination for determining a disease risk.

The present invention also provides a reagent for detecting an HLA-DP gene, comprising the one or more kinds of primers of the present invention. According to the reagent of the present invention, the method of the present invention can be easily performed.

The reagent of the present invention can contain the one or more kinds of primers of the present invention in a form of powder (e.g., lyophilization) or a solution. The solution is preferably an aqueous solution. Examples of the aqueous solution include water (e.g., sterile distilled water) and a buffer. Examples of the buffer include a TE (Tris-EDTA) buffer, a hydrochloric acid-potassium chloride buffer, a glycine-hydrochloric acid buffer, a citrate buffer, an acetate buffer, a citrate-phosphate buffer, a phosphate buffer, a Tris-hydrochloric acid buffer, a glycine-sodium hydroxide buffer, a carbonate-bicarbonate buffer, a borate buffer, and a tartrate buffer. When the reagent of the present invention is a solution containing the one or more kinds of primers of the present invention, a concentration of the primers in the solution varies depending on a factor such as use of the primers and a dilution ratio when the primers are used, but may be, for example, 0.1 to 100 mM, and preferably 1 to 10 mM. The solution may contain another component such as a stabilizer.

The present invention also provides a kit for detecting an HLA-DP gene, comprising (a) the one or more kinds of primers of the present invention and (b) a polymerase. According to the kit of the present invention, the method of the present invention can be easily performed.

As the polymerase, an appropriate polymerase according to the type of gene amplification method can be used. For example, in a case of PCR, a heat-resistant polymerase is preferably used, and in a case of LAMP, a strand displacement polymerase is preferably used. The polymerase is preferably a DNA polymerase.

The kit of the present invention may include an additional component in addition to (a) and (b). Examples of such a component include a deoxynucleoside triphosphate (dNTP) mixture, a reaction buffer, a molecular weight marker, and a control (e.g., standard materials of amplification products of various HLA alleles). When the kit of the present invention includes additional components, the components may be provided in a form in which the components are isolated from each other, for example, in a form in which the components are stored in different containers (e.g., tubes), but may be provided in a form in which the components are mixed in advance (e.g., PreMix) or the like.

In a certain embodiment, detection may be performed in real time (e.g., real time PCR). In this case, examples of a method for enabling detection in real time include an intercalator method and a fluorescent substance-labeled probe method. Thus, when detection in real time is intended, the kit of the present invention may further include a fluorescent substance or a fluorescent substance-labeled probe as an additional component. Examples of the fluorescent substance include a fluorescent substance (e.g., SYBR (registered trademark) Green I) used in an intercalator method. Examples of the fluorescent substance-labeled probe include a probe in which a fluorescent substance is linked to one of 5'-end and 3'-end and a quencher is linked to the other of the 5'-end and 3'-end (e.g., a TaqMan (registered trademark) probe).

### EXAMPLES

The present invention will be described in detail with reference to the following Examples, but the present invention is not limited to the following Examples.

Primer design procedures and evaluation for performing gene-specific PCR on an HLA-DPA1 gene and an HLA-DPB1 gene will be described.

### <Primer design>

Primers were designed (HLA-DPA1_v1, HLA-DPA1_v2, HLA-DPB1_v1, HLA-DPB1_v2, and HLA-DPB1_v3) by executing PCR primer design tool Primer 3 on regions stored in a human genome reference sequence (genome assembly version hg 38) and Alternate sequences (chr6_GL000250v2_alt, chr6_GL000251v2_alt, chr6_GL000252v2_alt, chr6_GL000253v2_alt, chr6_GL000254v2_alt, chr6_GL000255v2_alt, chr6_GL000256v2_alt, and chr6_GL383533v1_alt).

### <Confirmation of HLA allele comprehensiveness>

It was confirmed whether the designed primer set could comprehensively amplify HLA alleles by PCR *in silico* using publicly available whole genome sequence (WGS) data.

WGS data for 137 cases from HipSci Resource as published was downloaded from the European Nucleotide Archive (ENA, https://www.ebi.ac.uk/ena,study PRJEB 15299), and a FASTQ file including only reads that might be derived from an HLA gene and the vicinity thereof was created using BWA 0.7.17 and samtools 1.10.

HLA allele calling was performed using HLAHD 1.2.1 on the created FASTQ file of each sample (Tables 2 and 3).

**Table 2. 137 cases of HLA-DPA1 alleles registered in the HipSci Resource**

| # | HLA allele | Number of alleles |
|---|---|---|
| 1 | DPA1*01:03:01 | 222 |
| 2 | DPA1*02:01:01 | 26 |
| 3 | DPA1*02:01:02 | 13 |
| 4 | DPA1*02:02:02 | 4 |
| 5 | DPA1*02:06 | 3 |
| 6 | DPA1*01:08 | 2 |
| 7 | DPA1*02:07:01 | 2 |
| 8 | DPA1*01:03:05 | 1 |
| 9 | DPA1*02:01:04 | 1 |

**Table 3. 137 cases of HLA-DPB1 alleles registered in the HipSci Resource**

| # | HLA allele | Number of alleles |
|---|---|---|
| 1 | DPB1*04:01:01 | 122 |
| 2 | DPB1*02:01:02 | 33 |
| 3 | DPB1*04:02:01 | 29 |
| 4 | DPB1*03:01:01 | 21 |
| 5 | DPB1*01:01:01 | 16 |
| 6 | DPB1*11:01:01 | 8 |
| 7 | DPB1*06:01:01 | 6 |
| 8 | DPB1*02:02:01 | 5 |
| 9 | DPB1*13:01:01 | 5 |
| 10 | DPB1*14:01:01 | 5 |
| 11 | DPB1*10:01:01 | 4 |
| 12 | DPB1*16:01:01 | 4 |
| 13 | DPB1*05:01:01 | 3 |
| 14 | DPB1*15:01:01 | 3 |
| 15 | DPB1*20:01:01 | 2 |
| 16 | DPB1*09:01:01 | 2 |
| 17 | DPB1*17:01:01 | 2 |
| 18 | DPB1*19:01:01 | 2 |
| 19 | DPB1*02:01:04 | 1 |
| 20 | DPB1*33:01:01 | 1 |

For the created FASTQ file of each sample, a consensus sequences of HLA-DPA1 gene and HLA-DPB1 gene and a sequence in the vicinity thereof was created for each allele using freebayes 1.3.1, whatshap 1.0, and bcftools 1.10.2, and correlated with HLA allele information of each sample.

The designed primers were DPA1_Fw_v1, DPA1_Rv_v1, DPA1_Fw_v2, DPA1_Rv_v2, DPB1_Fw_v1, DPB1_Rv_v1, DPB1_Fw_v2, DPB1_Rv_v2, and DPB1_Fw_v3 (for details, see Table 4). It was evaluated whether a binding site to be a core sequence of the designed primer was present in the consensus sequences of 137 cases registered in HipSci Resource using Bowtie 2 2.3.5.1, and it was confirmed that the binding site was present in 99% or more of the consensus sequences.

### <Comparison of HLA allele comprehensiveness in HLA-DPB1 gene primer>

For known HLA-DPB1 gene primers (Patent Literature 1), comprehensiveness to the consensus sequences of 137 cases registered in HipSci Resource was evaluated using Bowtie 2 2.3.5.1. As a result, there were mismatches in 19 consensus sequences (mismatch: about 7%) among 137 cases (consensus sequence number: 274) including HLA alleles described in Table 3, and therefore it was considered that there was a high possibility that PCR amplification was not observed or uniform amplification was affected.

In contrast, each of the DPA1 and DPB1 gene primers designed as described above had a mismatch of less than 1% for 137 cases (consensus sequence number: 274). In particular, there was no mismatch in DPA1_Rv_v1, DPA1_Fw_v2, DPA1_Rv_v2, DPB1_Fw_v1, DPB1_Fw_v2, DPB1_Fw_v3, and DPB1_Rv_v2. Therefore, it was confirmed that the designed primers covered the HLA alleles described in Table 2 and Table 3.

### <Confirmation of specific PCR amplification by experiment>

In order to experimentally confirm whether the primer presented in Table 4 could specifically perform amplification by PCR, PCR reactions were performed using combinations of each primer with genomic DNA extracted from a cell line established from an African population (Daudi: D) or human peripheral blood mononuclear cells derived from Hispanic Americans (LP7:L) as a template. Nuclease-free Water (N) was used as a non-template control.

**Table 4. HLA-DPA1 gene and HLA-DPB1 gene primers**

| HLA gene | Name of primer | Primer sequence (5' → 3') | SEQ ID NO: |
|---|---|---|---|
| HLA-DPA1 | DPA1_Fw_v1 | A**AACCTATCCCCCTCCC**TCC | 2 |
| | DPA1_Rv_v1 | GCC**ATCTGACCTGGAAA**CCA | 4 |
| | DPA1_Fw_v2 | TTT**ACTGTACCCATGTC**CTTGGAG | 6 |
| | DPA1_Rv_v2 | CCT**CAGAACCCAGAAAGATT**CAGA | 8 |
| HLA-DPB1 | DPB1_Fw_v1 | AGAA**TCAGATGCTTTGAAGG**AGGT | 10 |
| | DPB1_Rv_v1 | GAGTCTT**GCCAGAGGTCTCATTA**A | 12 |
| | DPB1_Fw_v2 | TG**CGCCCATATTTTGAT**CTTGTTT | 14 |
| | DPB1_Rv_v2 | ATTGT**GAACTAGGGGACA**TTTGGA | 16 |
| | DPB1_Fw_v3 | CCA**AGAGAGGCAGTGTCG**TT | 18 |

| | | | |
|---|---|---|---|
| * The underlined bold portion indicates core sequence. For details, see <Identification of core sequence> described later. | | | |

### <PCR conditions for HLA-DPA1 gene>

For the PCR reaction, using KOD One PCR Master Mix (Dye-free 2 x PCR Master Mix) (Toyobo Co., Ltd.), a total of 25 µL solution containing 12.5 µL of KOD One PCR Master Mix (Dye-free 2 x PCR Master Mix), 3.75 µL of a forward primer (2 µM), 3.75 µL of a reverse primer (2 µM), 2.5 µL of Nuclease-free Water, and 2.5 µL of a genomic DNA solution (5 ng) was prepared. A process including two steps consisting of a step of keeping this solution at 94°C for 15 seconds and a step of keeping the solution at 68°C for three minutes was repeatedly performed 35 times. Note that, for this PCR, C1000 Touch Thermal Cycler (Bio-Rad Laboratories, Inc.) was used.

### <PCR conditions for HLA-DPB1 gene>

For the PCR reaction, using SeqAmp DNA polymerase (Takara Bio Inc.), a total of 20 µL solution containing 10.0 µL of 2X SeqAmp PCR Buffer (includes Mg²⁺, dNTPs), 2 µL of a forward primer (2 µM), 2 µL of a reverse primer (2 µM), 3.1 µL of Nuclease-free Water, 0.4 µL of NSeqAmp DNA polymerase, and 2.5 µL of a genomic DNA solution (12.5 ng) was prepared. This solution was kept at 94°C for one minute. Subsequently, a process including two steps consisting of a step of keeping the solution at 98°C for 10 seconds and a step of keeping the solution at 68°C for 15 minutes was repeatedly performed 30 times. Note that, for this PCR, C1000 Touch Thermal Cycler (Bio-Rad Laboratories, Inc.) was used.

Next, after the PCR reaction, a PCR amplification product was purified using Sera-Mag Select (Cytiva), and eluted with 25 µL of Buffer EB (QIAGEN).

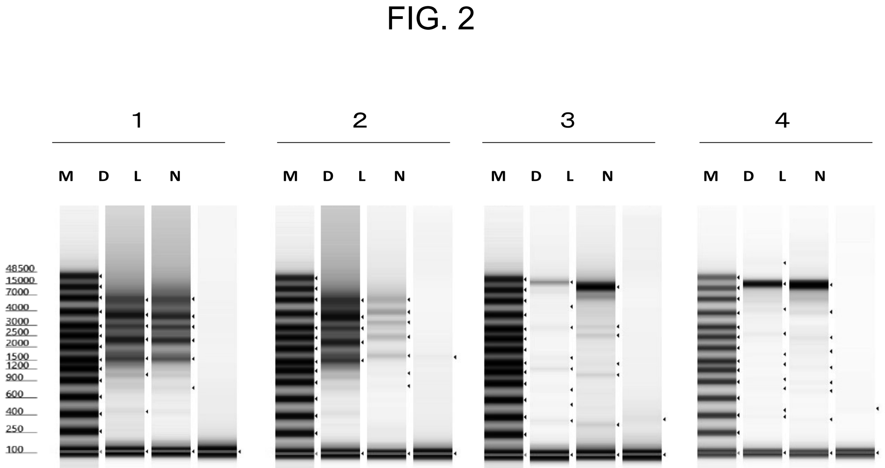

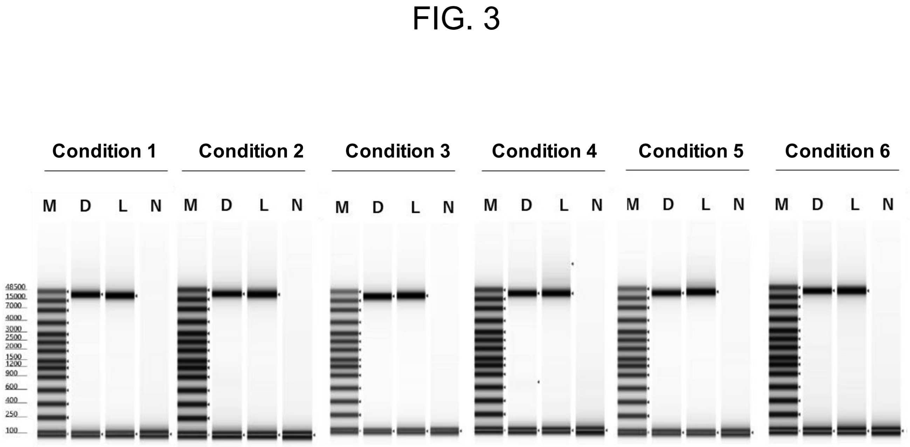

The PCR amplification product after purification was subjected to electrophoresis using Genomic DNA ScreenTape system (Agilent Technologies, Inc.), and a status of PCR amplification was confirmed (FIGS. 2 and 3). Amplification conditions (primer set) are as follows.

**Table 5. Amplification conditions of HLA-DPA1 gene (primer set)**

| Condition | Forward primer | Reverse primer |
|---|---|---|
| 1 | DPA1_Fw_v1 | DPA1_Rv_v1 |
| 2 | | DPA1_Rv_v2 |
| 3 | DPA1_Fw_v2 | DPA1_Rv_v1 |
| 4 | | DPA1_Rv_v2 |

**Table 6. Amplification conditions of HLA-DPB1 gene (primer set)**

| Condition | Forward primer | Reverse primer |
|---|---|---|
| 1 | DPB1_Fw_v1 | DPB1_Rv_v1 |
| 2 | | DPB1_Rv_v2 |
| 3 | DPB1_Fw_v2 | DPB1_Rv_v1 |
| 4 | | DPB1_Rv_v2 |
| 5 | DPB1_Fw_v3 | DPB1_Rv_v1 |
| 6 | | DPB1_Rv_v2 |

### <Sequencing using long read sequencer>

The PCR amplification products were sequenced using Sequel system (Pacific Biosciences of California, Inc.).

PCR amplification was performed using the primer set of SEQ ID NO:6 and SEQ ID NO:8, the primer set of SEQ ID NO:10 and SEQ ID NO:12, and the primer set of SEQ ID NO:14 and SEQ ID NO:16 in which universal sequence was added to the 5'-end of the 5'-amino-modified primer, as shown in Table 4, and setting of a measuring instrument, reaction conditions, and the addition amount of a reagent were performed basically according to Procedure & Checklist Part Number 101-791-700 version 02 provided by PacBio Inc. using SMRTbell Express Template Prep Kit 2.0. Note that the universal sequence is a sequence described in Procedure & Checklist Part Number 101-791-800 provided by PacBio Inc., and /5AmMC6/gcagtcgaacatgtagctgactcaggtcac (SEQ ID NO:21) was added to a forward primer, and /5AmMC6/tggatcacttgtgcaagcatcacatcgtag (SEQ ID NO:22) was added to a reverse primer.

### <PCR conditions>

For the PCR reaction, using KOD One PCR Master Mix (Dye-free 2 x PCR Master Mix) (Toyobo Co., Ltd.), a total of 25 µL solution containing 12.5 µL of KOD One PCR Master Mix (Dye-free 2 x PCR Master Mix), 3.75 µL of a forward primer (2 µM), 3.75 µL of a reverse primer (2 µM), and 5 µL of a genomic DNA solution (10 ng) was prepared. This solution was kept at 94°C for one minute. Subsequently, a process including two steps consisting of a step of keeping the solution at 98°C for 10 seconds and a step of keeping the solution at 68°C for three minutes was repeatedly performed 30 times. Note that, for this PCR, C1000 Touch Thermal Cycler (Bio-Rad Laboratories, Inc.) was used.

For the consensus sequence obtained by the Sequel system, HLA allele typing was performed using an IMGT/HLA database registration sequence as a reference sequence.

Genomic DNAs extracted from a cell line established from an African population (Daudi) and a cell line established from a Japanese population were used as templates for analysis. Using the primer set of SEQ ID NO:6 and SEQ ID NO:8 and the primer set of SEQ ID NO:10 and SEQ ID NO:12 described in Table 4, 37 cases of cell lines established from the Japanese population were analyzed, and using the primer set of SEQ ID NO:14 and SEQ ID NO:16 described in Table 4, 28 cases of cell lines established from the Japanese population were analyzed.

As a result, it was confirmed that the primer sets selected this time could highly comprehensively amplify the HLA allele group (Tables 7, 8, and 9).

**Table 7. HLA-DPA1 alleles analyzed for 37 cases of cell lines established from Japanese population by using SEQ ID NO:6 and SEQ ID NO:8 described in Table 4**

| # | HLA allele | Number of alleles |
|---|---|---|
| 1 | DPA1*01:03:01:01 | 19 |
| 2 | DPA1*01:03:01:03 | 2 |
| 3 | DPA1*01:03:01:05 | 2 |
| 4 | DPA1*01:03:01:15 | 5 |
| 5 | DPA1*02:01:01:02 | 6 |
| 6 | DPA1*02:01:01:03 | 1 |
| 7 | DPA1*02:01:01:14 | 1 |
| 8 | DPA1*02:01:01:17 | 1 |
| 9 | DPA1*02:02:02:01 | 20 |
| 10 | DPA1*02:02:02:02 | 3 |
| 11 | DPA1*02:02:02:10 | 4 |

**Table 8. HLA-DPB1 alleles analyzed for 37 cases of cell lines established from Japanese population by using SEQ ID NO:10 and SEQ ID NO:12 described in Table 4**

| # | HLA allele | Number of alleles |
|---|---|---|
| 1 | DPB1*02:01:02:01 | 10 |
| 2 | DPB1*02:01:02:05 | 2 |
| 3 | DPB1*02:01:02:11 | 1 |
| 4 | DPB1*02:01:02:20 | 1 |
| 5 | DPB1*02:01:02:32 | 1 |
| 6 | DPB1*02:01:02:38 | 1 |
| 7 | DPB1*02:02:01:02 | 2 |
| 8 | DPB1*02:02:01:07 | 1 |
| 9 | DPB1*03:01:01:08 | 2 |
| 10 | DPB1*03:01:01:09 | 3 |
| 11 | DPB1*04:01:01:26 | 5 |
| 12 | DPB1*04:02:01:02 | 6 |
| 13 | DPB1*05:01:01:01 | 13 |
| 14 | DPB1*05:01:01:03 | 3 |
| 15 | DPB1*05:01:01:04 | 2 |
| 16 | DPB1*05:01:01:05 | 1 |
| 17 | DPB1*05:01:01:08 | 3 |
| 18 | DPB1*05:01:01:11 | 2 |
| 19 | DPB1*06:01:01:01 | 1 |
| 20 | DPB1*09:01:01 | 5 |
| 21 | DPB1*14:01:01:01 | 1 |
| 22 | DPB1*17:01:01:01 | 1 |
| 23 | DPB1*35:01:01 | 1 |

**Table 9. HLA-DPB1 alleles analyzed for 28 cases of cell lines established from Japanese population by using SEQ ID NO:14 and SEQ ID NO:16 described in Table 4**

| # | HLA allele | Number of alleles |
|---|---|---|
| 1 | DPB1*02:01:02:01 | 4 |
| 2 | DPB1*02:01:02:11 | 1 |
| 3 | DPB1*02:01:02:32 | 2 |
| 4 | DPB1*02:02:01:02 | 5 |
| 5 | DPB1*03:01:01:01 | 2 |
| 6 | DPB1*03:01:01:09 | 1 |
| 7 | DPB1*04:01:01:26 | 6 |
| 8 | DPB1*04:02:01:02 | 6 |
| 9 | DPB1*05:01:01:01 | 13 |
| 10 | DPB1*05:01:01:04 | 3 |
| 11 | DPB1*09:01:01:01 | 10 |
| 12 | DPB1*14:01:01:01 | 1 |

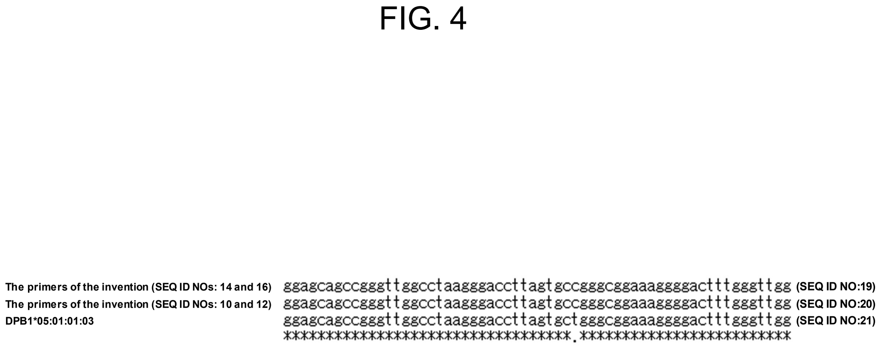

As a result, it was confirmed that it was possible to amplify an HLA allele deduced to be novel, which is different from DPB1*05:01:01:03 by one nucleotide, by using the primer set selected this time (FIG. 4).

It was confirmed that the primer set selected this time could analyze full-length genes of HLA-DPA1 gene and HLA-DPB1 gene comprising an untranslated region thereof, and the HLA-DP region could be phased for each allele (Table 10).

**Table 10. Allele typing and phasing of HLA-DPA1 gene and HLA-DPB1 gene by long read sequence**

| Sample | Allele | HLA-DPA1 | HLA-DPB1 | rs9277534 |
|---|---|---|---|---|
| Daudi | Allele 1 | DPA1*01:03:01:35 | DPB1*02:01:02:18 | A |
| | Allele 2 | DPA1*02:01:01:02 | DPB1*106:01 | A |
| Cell lines established from Japanese population | Allele 1 | DPA1*01:03:01:01 | DPB1*09:01:01 | G |
| | Allele 2 | DPA1*02:01:01:02 | DPB1*02:01:02:11 | A |

### <Identification of core sequence>

In order to identify core sequences of forward primers or reverse primers, it was confirmed whether the sequences completely matched the human genome reference sequence (genome assembly version hg 38) by shortening nucleotides one by one for the length of the designed primer from each of 5'-side and 3'-side, and then core sequences that uniquely matched in an upstream and downstream of target genes were identified (the underlined bold sequences in Table 4). For the core sequence, it was confirmed that the binding sites of all primers uniquely matched 99% or more with the consensus sequences of the target genes in 137 cases from the HipSci Resource.

[SEQUENCE LISTING]

## Claims

1. One or more kinds of HLA-DP gene primers selected from the group consisting of the following (1) and (2), and a combination thereof:
(1) one or more kinds of HLA-DPA1 gene primers, which are
(1a) a first primer comprising a nucleotide sequence of SEQ ID NO:1 or SEQ ID NO:5, or a nucleotide sequence complementary thereto and/or (1b) a second primer comprising a nucleotide sequence of SEQ ID NO:3 or SEQ ID NO:7, or a nucleotide sequence complementary thereto; and
(2) one or more kinds of HLA-DPB1 gene primers, which are
(2a) a first primer comprising a nucleotide sequence of SEQ ID NO:9, SEQ ID NO:13, or SEQ ID NO:17, or a nucleotide sequence complementary thereto and/or (2b) a second primer comprising a nucleotide sequence of SEQ ID NO:11 or SEQ ID NO:15, or a nucleotide sequence complementary thereto.

2. The one or more kinds of HLA-DP gene primers according to claim 1, which are an HLA-DP gene amplification primer set selected from the group consisting of the following (1') and (2'), and a combination thereof:
(1') an HLA-DPA1 gene amplification primer set, which is
a primer set comprising (1a) a first primer comprising a nucleotide sequence of SEQ ID NO:1 or SEQ ID NO:5, or a nucleotide sequence complementary thereto, and (1b) a second primer comprising a nucleotide sequence of SEQ ID NO:3 or SEQ ID NO:7, or a nucleotide sequence complementary thereto; and
(2') an HLA-DPB1 gene amplification primer set, which is
a primer set comprising (2a) a first primer comprising a nucleotide sequence of SEQ ID NO:9, SEQ ID NO:13, or SEQ ID NO:17, or a nucleotide sequence complementary thereto, and (2b) a second primer comprising a nucleotide sequence of SEQ ID NO:11 or SEQ ID NO:15, or a nucleotide sequence complementary thereto.

3. The one or more HLA-DP gene primers according to claim 1, wherein
(1a) the first primer comprising a nucleotide sequence of SEQ ID NO:1 or SEQ ID NO:5, or a nucleotide sequence complementary thereto is (1a') a first primer comprising a nucleotide sequence of SEQ ID NO:2 or SEQ ID NO:6, or a nucleotide sequence complementary thereto,
(1b) the second primer comprising a nucleotide sequence of SEQ ID NO:3 or SEQ ID NO:7, or a nucleotide sequence complementary thereto is (1b') a second primer comprising a nucleotide sequence of SEQ ID NO:4 or SEQ ID NO:8, or a nucleotide sequence complementary thereto,
(2a) the first primer comprising a nucleotide sequence of SEQ ID NO:9, SEQ ID NO:13, or SEQ ID NO:17, or a nucleotide sequence complementary thereto is (2a') a first primer comprising a nucleotide sequence of SEQ ID NO:10, SEQ ID NO:14, or SEQ ID NO:18, or a nucleotide sequence complementary thereto, and
(2b) the second primer comprising a nucleotide sequence of SEQ ID NO:11 or SEQ ID NO:15, or a nucleotide sequence complementary thereto is (2b') a second primer comprising a nucleotide sequence of SEQ ID NO:12 or SEQ ID NO:16, or a nucleotide sequence complementary thereto.

4. A method for detecting an HLA-DP gene, comprising:
detecting one or more kinds of HLA-DP genes in a sample obtained from a human subject using the one or more kinds of HLA-DP gene primers according to any one of claims 1 to 3,
wherein the one or more kinds of HLA-DP genes are selected from the group consisting of an HLA-DPA1 gene, an HLA-DPB1 gene, and a combination thereof.

5. The method according to claim 4, wherein the one or more kinds of HLA-DP genes are detected by amplifying the one or more kinds of HLA-DP genes.

6. A reagent for detecting an HLA-DP gene, comprising the one or more kinds of HLA-DP gene primers according to any one of claims 1 to 3.

7. A kit for detecting an HLA-DP gene, comprising:
(a) the one or more kinds of HLA-DP gene primers according to any one of claims 1 to 3; and
(b) a polymerase.
